Europäisches Patentamt

European Patent Office

Office européen des brevets

(19)

(11) Veröffentlichungsnummer: **0 006 151**
**B1**

(12) # EUROPÄISCHE PATENTSCHRIFT

(45) Veröffentlichungstag der Patentschrift:
22.07.81

(21) Anmeldenummer: 79101624.9

(22) Anmeldetag: 28.05.79

(51) Int. Cl.³: **C 07 D 471/22** // (C07D471/22, 221/00, 221/00, 221/00, 221/00)

(54) Verfahren zur Herstellung von 17-Hydroxyspartein.

(30) Priorität: 08.06.78 DE 2825117

(43) Veröffentlichungstag der Anmeldung:
09.01.80 Patentblatt 80/1

(45) Bekanntmachung des Hinweises auf die Patenterteilung:
22.07.81 Patentblatt 81/29

(84) Benannte Vertragsstaaten:
AT BE CH DE FR GB IT LU NL SE

(56) Entgegenhaltungen:
DE-A-2 360 475
Chemical Abstracts, Band 51, Nr. 11,
10. Juni 1957,
COLUMBUS, OHIO, USA,
M. RINK et al.: »17-hydroxysparteine«
Spalte 8114 DE
Chemical Abstracts, Band 78, Nr. 11,
19. März 1973,
COLUMBUS, OHIO, USA,
J. SKOLIK et al.: »Deuteroderivatives of quinolizidine alkaloids. I. Synthesis and infrared spectral
analysis of carbon-17 deuteroderivatives of sparteine«

(73) Patentinhaber: Kali-Chemie Pharma GmbH,
Hans-Böckler-Allee 20 Postfach 220,
D-3000 Hannover 1 (DE)

(72) Erfinder: Hachmeister, Bernd, Dipl.-Chem., Dr. rer.nat.,
Laurinweg 18a, D-3000 Hannover (DE)

(74) Vertreter: Lauer, Dieter, Dr., c/o Kali-Chemie
Aktiengesellschaft Postfach 220, D-3000 Hannover 1 (DE)

Seite 500, Spalte 1, Abstract Nr. 72 405 U
Chemical Abstracts, Band 78, Nr. 21,
28. Mai 1973,
COLUMBUS, OHIO, USA,
K. FURUYA et al.: »Microbial transformation.
XXVI. Microbial oxidation of (—)sparteine«
Seite 240, Spalte 2, Abstract, Nr. 134 443 R

## Verfahren zur Herstellung von 17-Hydroxyspartein

Die Erfindung betrifft ein Verfahren zur Herstellung von 17-Hydroxyspartein durch Oxydation von Spartein.

Nachdem in neuerer Zeit vorteilhafte pharmakologische Wirkungen verschiedener Sparteinderivate bekanntgeworden sind (DE-A 23 60 475), zu deren Herstellung von 17-Hydroxyspartein ausgegangen wird, ist ein Bedürfnis nach einem technisch und wirtschaftlich vernünftigen Verfahren zur Herstellung von 17-Hydroxyspartein entstanden.

Ein bekanntes Verfahren (M. Rink, K. Grabowski, Arch. Pharm., 61 [1956], 695) oxydiert Spartein mittels Chromtrioxid. Bei der Aufarbeitung der Reaktionsmischung fallen erhebliche Mengen wäßriger Lösungen von $Chrom^{III}$- und $Chrom^{VI}$-Salzen an, aus denen die sehr giftigen Chromverbindungen nur durch aufwendige Verfahren abgetrennt werden können. Außerdem beträgt die Ausbeute an 17-Hydroxyspartein nur 39%.

Daneben ist noch die Oxydation von Spartein zu 17-Hydroxyspartein unter Zuhilfenahme des Mikroorganismus Trametes gibbosa bekannt (K. Furuya, K. Aida, Y. Koiso, S. Okuda, Chem. Pharm. Bull., 21 [1973], 231). Dieses Verfahren arbeitet mit großen Flüssigkeitsmengen, ist dementsprechend aufwendig und liefert das gewünschte 17-Hydroxyspartein in der ebenfalls vergleichsweise bescheidenen Ausbeute von nur 38%.

Lediglich akademischen Charakter hat die Oxydation mit dem System Ascorbinsäure-$Eisen^{II}$-Sauerstoff (F. Jaminet, J. pharm. Belg., 13 [1958], 577), die sehr zeitaufwendig ist, und nach mehrtägiger Reaktion nur einen Teil des eingesetzten Sparteins umsetzt.

Demgegenüber liegt der Erfindung die Aufgabe zugrunde, ein Verfahren zur Herstellung von 17-Hydroxyspartein aus Spartein bereitzustellen, das die Nachteile der bekannten Verfahren vermeidet, insbesondere das 17-Hydroxyspartein in hoher Ausbeute gewinnen läßt, einfach in der Verfahrensführung ist und giftige und nur schwierig abzutrennende Reaktionspartner vermeidet.

Von besonderer Bedeutung bei der Lösung dieser Aufgabe ist dabei die in der älteren Literatur (R. Willstätter, W. Marx, Chem. Ber., 38 [1905], 1773; R. Willstätter, E. Fourneau, Chem. Ber., 35 [1902], 1912) beschriebene Beobachtung, daß Spartein in schwefelsaurer Lösung gegen Permanganat beständig ist.

Entgegen dieser Beobachtung wurde nun überraschenderweise gefunden, daß sich 17-Hydroxyspartein bildet, wenn man Spartein mit Permanganat in wäßrig saurer Lösung bei einem pH von 0 bis 5 oxidiert.

Dieser gegensätzliche Befund ist auch deswegen überraschend, weil Permanganat an sich übliches Oxidationsmittel ist und andere ebenfalls übliche Oxidationsmittel wie $Quecksilber^{II}$-acetat, $Kaliumcyanoferrat^{III}$ oder Hypobromit zu anderen Produkten, beispielsweise 5,6-Didehydrospartein, 5,6,11,12-Tetradehydrospartein, 17-Oxospartein bzw. zu dimeren Sparteinen, führen.

Gegenstand der Erfindung ist demnach ein Verfahren zur Herstellung von 17-Hydroxyspartein, wobei dem Verfahren die bekannte, aber erfolglose Behandlung von Spartein mit Permanganat in saurer Lösung zugrunde liegt und das erfinderische Neue dadurch gekennzeichnet ist, daß man Spartein mit Permanganat in wäßrig saurer Lösung bei einem pH von 0 bis 5 oxidiert.

Vorteilhafterweise oxidiert man bei einem mäßig sauren pH von von 2 bis 3,5.

Zur Einstellung und Aufrechterhaltung der sauren Reaktion während der erfindungsgemäßen Oxydation kann an sich jede beliebige, insbesondere anorganische Säure, beispielsweise Salz- oder Schwefelsäure, verwendet werden. Vorteilhafterweise arbeitet man in schwefelsaurer Lösung.

Der Temperaturbereich, in dem die Oxydation ausgeführt wird, ist nicht besonders kritisch. Man kann im Temperaturbereich von 0 bis 100° C arbeiten; die Erfindung bevorzugt den Bereich von etwa 0 bis 20° C.

Als die Oxydation bewirkendes Permanganat kann jedes gängige, wasserlösliche Permanganatsalz eingesetzt werden, bevorzugt das Kaliumpermanganat. Im Gegensatz zur Chromsäureoxydation, wo das entstehende 17-Hydroxyspartein als Chromat-Salz ausgefällt und als solches isoliert wird, und daher ein entsprechender Überschuß des Oxydationsmittels eingesetzt werden muß, ist bei der Oxydation mittels Permanganat ein nur geringer Überschuß an Oxydationsmittel erforderlich. Nach beendigter Oxydation liegt alles Mangan als $Mn^{II}$ vor, das leicht als wasserunlösliches Salz ausgefällt und abgetrennt werden kann, bevorzugt als Manganammoniumphosphat, was durch bloßes Zufügen entsprechender Mengen wasserlöslicher Ammonium- und Phosphat-Verbindungen nach beendigter Oxydation und Einstellung der Reaktionsmischung auf neutrale bis schwach alkalische Reaktion bewerkstelligt werden kann. Das entstandene 17-Hydroxyspartein bleibt als Salz in Lösung und kann daraus nach Abtrennung der Mangankomponente in üblicher Weise extrahiert werden.

Die Reaktion nach dem erfindungsgemäßen Verfahren verläuft mit hoher Selektivität und liefert das gewünschte 17-Hydroxyspartein in hoher Ausbeute von über 90%. Die Oxydation läuft schnell ab und ist nach ca. längstens einer Stunde beendet. Die Aufarbeitung ist einfacher als bei der Chromsäureoxydation, da sich das entstehende $Mn^{II}$-Salz leicht abtrennen läßt. Die Reaktion ist einfacher zu beherrschen als die Chromsäureoxydation, die im Gegensatz zur erfindungsgemäßen Oxydation mit einer sehr stark exothermen Reaktion einsetzt.

Zusammengefaßt erfolgt die Herstellung von 17-Hydroxyspartein erfindungsgemäß derart, daß man zu einer wäßrigen Lösung von Sparteinsalz festes Permanganat oder eine Permanganatlösung hinzufügt. Man arbeitet im Temperaturbereich zwischen 0 und 100°C, vorzugsweise zwischen 0 und 20°C, im sauren Bereich von pH 0 bis 5, vorzugsweise bei einem pH zwischen 2 und 3,5. Das sich im Laufe der Reaktion bildende Mangan$^{II}$ wird im schwach alkalischen Bereich als Manganammoniumphosphat kristallin ausgefällt und von der wäßrigen Lösung abgetrennt, in der die Produkt-Base als Salz gelöst bleibt. Das 17-Hydroxyspartein kann im stark alkalischen pH-Bereich aus der wäßrigen Lösung mit den üblichen organischen Extraktionsmitteln ausgeschüttelt werden.

Das nachfolgende Beispiel dient der näheren Erläuterung der Erfindung:

## Beispiel

Eine Lösung von 10,5 g Kaliumpermanganat in 200 ml Wasser wird zu einer eisgekühlten Lösung von 42,2 g Sparteinsulfat-Pentahydrat in 40 ml Wasser gegeben. Die Zulaufgeschwindigkeit wird so reguliert, daß die Innentemperatur 20°C nicht überschreitet. Durch gleichzeitige Zugabe von Schwefelsäure wird der pH-Wert zwischen 2 und 3,5 gehalten. Nach 40 Minuten wird die Lösung kurz auf 40°C erwärmt, wobei restlicher Braunstein in Lösung geht. Der klaren Lösung werden nacheinander 5,4 g Ammoniumchlorid und 35,8 g di-Natriumhydrogenphosphat 12 H$_2$O zugesetzt. Ammoniummanganphosphat wird durch Zugabe von 15 ml 40%iger Natronlauge auskristallisiert, anschließend abgesaugt und mit wenig Wasser gewaschen. Das Filtrat wird mit Natronlauge stark alkalisch gemacht und mit Methylenchlorid extrahiert. Nach Trocknen der organischen Phase und Abdestillieren des Extraktionsmittels im Vakuum bleiben 23,8 g (=94,8% d. Th.) 17-Hydroxyspartein als Öl zurück.

Dieses Öl kann ohne weitere Reinigung weiter umgesetzt werden, beispielsweise mit Grignard-Verbindungen zu 17-Alkylspartein gemäß DE-A 23 60 475.

Wird die organische Phase in üblicher Weise mit Aktivkohle geklärt, so bleiben nach Abfiltrieren der Aktivkohle und Einengen der Lösung 22,6 g (=90% d. Th.) hellgelbes Öl zurück, das nach längerem Stehen kristallisiert. Die Kristalle schmelzen bei 72—75°C.

## Patentansprüche

1. Verfahren zur Herstellung von 17-Hydroxyspartein durch Oxydation von Spartein in saurer Lösung, dadurch gekennzeichnet, daß man Spartein mit Permanganat in wäßrig saurer Lösung bei einem pH von 0 bis 5 oxidiert.

2. Verfahren nach Anspruch 1, dadurch gekennzeichnet, daß man bei einem pH von von 2 bis 3,5 oxidiert.

3. Verfahren nach Anspruch 1 oder 2, dadurch gekennzeichnet, daß man in schwefelsaurer Lösung oxidiert.

4. Verfahren nach einem der Ansprüche 1 bis 3, dadurch gekennzeichnet, daß man im Temperaturbereich von 0 bis 100°C oxidiert.

5. Verfahren nach Anspruch 4, dadurch gekennzeichnet, daß man im Temperaturbereich von 0 bis 20°C oxidiert.

6. Verfahren nach einem der Ansprüche 1 bis 5, dadurch gekennzeichnet, daß man mit Kaliumpermanganat oxidiert.

7. Verfahren nach einem der Ansprüche 1 bis 6, dadurch gekennzeichnet, daß man das sich bildende Mn$^{II}$ als wasserunlösliches Salz aus der Reaktionsmischung ausfällt und abtrennt.

8. Verfahren nach Anspruch 7, dadurch gekennzeichnet, daß man das sich bildende Mn$^{II}$ als Manganammoniumphosphat ausfällt.

## Claims

1. Process for the production of 17-hydroxysparteine by oxidation of sparteine in acid solution, characterised in that sparteine is oxidised with permanganate in aqueous acid solution at a pH of 0 to 5.

2. Process according to claim 1, characterised in that the oxidation is effected at a pH of 2 to 3.5.

3. Process according to claim 1 or 2 characterised in that oxidation is effected in a sulphuric acid solution.

4. Process according to one of claims 1 to 3, characterised in that oxidation is effected in a temperature range of 0 to 100°C.

5. Process according to claim 4, characterised in that oxidation is effected in a temperature range of 0 to 20°C.

6. Process according to one of claims 1 to 5, characterised in that oxidation is effected with potassium permanganate.

7. Process according to one of claims 1 to 6, characterised in that the Mn$^{II}$ which forms is precipitated out of the reaction mixture as water-insoluble salt and separated.

8. Process according to claim 7 characterised in that the Mn$^{II}$ which forms is precipitated as a manganese ammonium phosphate.

## Revendications

1. Procédé de préparation de 17-hydroxyspartéine par oxydation de spartéine en solution acide, caractérisé en ce qu'on oxyde de la spartéine avec du permanganate en solution acide aqueuse à un pH de 0 à 5.

2. Procédé suivant la revendication 1, caractérisé en ce qu'on effectue l'oxydation à un pH de 2 à 3,5.

3. Procédé suivant la revendication 1 ou 2, caractérisé en ce qu'on effectue l'oxydation dans une solution d'acide sulfurique.

4. Procédé suivant une des revendications 1 à 3, caractérisé en ce qu'on effectue l'oxydation à une température se situant dans l'intervalle allant de 0 à 100°C.

5. Procédé suivant la revendication 4, caractérisé en ce qu'on effectue l'oxydation à une température se situant dans l'intervalle allant de 0 à 20°C.

6. Procédé suivant une des revendications 1 à 5, caractérisé en ce qu'on effectue l'oxydation avec du permanganate de potassium.

7. Procédé suivant une des revendications 1 à 6, caractérisé en ce qu'on précipite et sépare, du mélange réactionnel, le Mn(II) qui se forme, sous forme d'un sel insoluble dans l'eau.

8. Procédé suivant la revendication 7, caractérisé en ce qu'on précipite le Mn(II) qui se forme, sous forme de phosphate de manganèse-ammonium.